# EUROPEAN PATENT SPECIFICATION

(11) **EP 4 059 524 B1**
(45) Date of publication and mention of the grant of the patent: **12.02.2025**
(21) Application number: 21164647.6
(22) Date of filing: 24.03.2021
(51) Int. Cl.: A61L 9/20, A61L 2/10, A61L 2/24

(54) **METHOD OF SELF-DISINFECTION OF MOBILE ROBOT**
VERFAHREN ZUR SELBSTDESINFEKTION EINES MOBILEN ROBOTERS
PROCÉDÉ D'AUTODÉSINFECTION D'UN ROBOT MOBILE

(30) Priority: 15.03.2021 US 202117202149
(43) Date of publication of application: 21.09.2022
(73) Proprietor: Blue Ocean Robotics Aps, 5260 Odense (DK)
(72) Inventor: ØSTERGAARD, John Erland, 5260 Odense (DK); VITZRABIN, Efraim, 5260 Odense (DK); CHAWLA, Rajat, 5260 Odense (DK)
(74) Representative: Cross, James Peter Archibald

(56) References cited:
- CN-A- 111 546 359
- KR-A- 20150 073 616

## Description

### BACKGROUND

Mobile devices, such as mobile robots, can be operated so as to disinfect indoor areas, such as a room, that have an unclean surfaces. Typically, such devices do not disinfect an area in an efficient manner, and may fail to disinfect all contaminated surfaces.
Moreover, the mobile robots can have contaminated surfaces, which contaminate the area that the mobile robot is cleaning, or other areas, as the robot moves.

KR 2015 0073616 A concerns a robot cleaner having an ultraviolet light emitting diode.

### BRIEF SUMMARY

Aspects of the invention are defined in the accompanying claims. According to a first aspect there is provided a method in accordance with claim 1. Preferred optional features are defined in the dependent claims.

According to an implementation of the disclosed subject matter, a method may be provided that includes moving a mobile robot in a path within a predetermined area using a drive system of the mobile robot, and outputting ultraviolet (UV) light from a first light source onto a portion of at least one of a plurality of wheels of the drive system based on a first dosage level.

Additional features, advantages, and implementations of the disclosed subject matter may be set forth or apparent from consideration of the following detailed description, drawings, and claims. Moreover, it is to be understood that both the foregoing summary and the following detailed description are illustrative and are intended to provide further explanation without limiting the scope of the claims.

### BRIEF DESCRIPTION OF THE DRAWINGS

The accompanying drawings, which are included to provide a further understanding of the disclosed subject matter, are incorporated in and constitute a part of this specification. The drawings also illustrate implementations of the disclosed subject matter and together with the detailed description serve to explain the principles of implementations of the disclosed subject matter. No attempt is made to show structural details in more detail than may be necessary for a fundamental understanding of the disclosed subject matter and various ways in which it may be practiced.
FIG. 1 shows an example method of moving a mobile robot and outputting UV light according to an implementation of the disclosed subject matter.

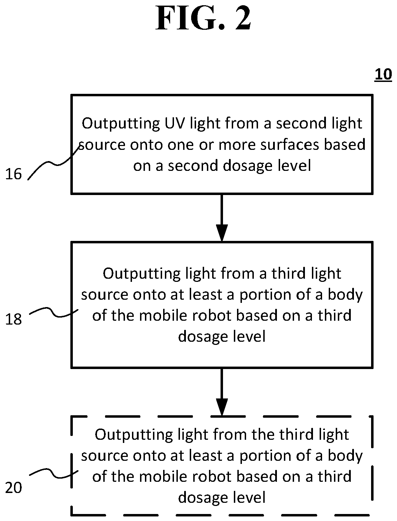

FIG. 2 shows the example method of FIG. 1 may include outputting light from a second light source according to an implementation of the disclosed subject matter.
FIGS. 3-5 show a plurality of external views of the mobile robot having sensors to detect surfaces and objects in an area, and light sources to output UV light according to implementations of the disclosed subject matter.
FIGS. 6A-6D show a portion of the drive system of the mobile robot, including wheels and light sources to disinfect the wheels and/or other surfaces according to implementations of the disclosed subject matter.
FIG. 7 shows the mobile robot that includes a UV light source coupled to an arm according to an implementations of the disclosed subject matter.
FIGS. 8A-8B show example paths of the mobile robot to apply a dosage of UV light in an area according to an implementation of the disclosed subject matter.
FIG. 9 shows an example of the mobile robot outputting UV light onto surfaces, objects, hotspots, and/or reference tags of an area to disinfect them according to an implementation of the disclosed subject matter.
FIG. 10 shows an example configuration of the mobile robot of FIGS. 3-7 according to an implementation of the disclosed subject matter.
FIG. 11 shows a network configuration which may include a plurality of mobile robots according to implementations of the disclosed subject matter.

### DETAILED DESCRIPTION

device mobile robot may be used to disinfect a predetermined area such as a room, a building, surfaces, air, objects, or the like in an environment using ultraviolet (UV) light from a light source. The mobile robot may also disinfect itself, such as its wheels and body, using UV radiation from light sources. Different dosages of UV light may be applied to the surfaces, air, objects, and to the mobile robot.

In some implementations, the mobile robot may autonomously enter a predetermined area, and may output UV light at a predetermined dosage level to disinfect the area. The mobile robot may have a map of the area stored in memory, may receive the map via a network interface, and/or may map the area using one or more sensors. The actuated mobile robot may receive a path via the network interface and/or determine a path to move within the area and to disinfect the area by outputting UV light from the light source. The path may be selected and/or determined so as to minimize the amount of time to apply the dosage level of UV light and disinfect the area. The mobile robot may disinfect its wheels as the mobile robot moves along the path, and/or when the mobile robot is stationary. The mobile robot may disinfect its body with a UV light source to prevent spreading contaminants to different areas.

Progress of applying the dosage level of UV light may be monitored by generating an exposure plot of the portions of the area that have been disinfected. In some implementations, the mobile robot may determine and/or detect portions of the area that have not received the dosage of UV light. For such portions, the mobile robot may adjust an arm with another light source to output UV light to the portion of the area, and/or onto the body of the mobile robot. The mobile robot may also disinfect at least a portion of one or more wheels that may have contacted one or more surfaces of the area.

The mobile robot may be used as part of a regular cleaning cycle of a room, building, or the like, and may prevent and/or reduce the spread of infectious diseases, viruses, bacteria, and other types of harmful organic microorganisms in the environment by breaking down their DNA-structure with UV light. The mobile robot may reduce human error in cleaning an area, room, building, or the like by tracking the location and/or intensity (e.g., optical power of UV light) of light radiated, and determine which areas may need to be radiated and/or cleaned. The mobile robot may prevent and/or reduce the spread of infectious diseases, viruses, bacteria, and other types of harmful organic microorganisms by using UV light to clean the wheels and/or body of the mobile robot.

The mobile robot may be operated manually, autonomously, and/or may receive control signals to control the movement of the mobile robot with a room, building, area, or the like when operating in a tele-operation mode.

Traditional disinfection methods and devices using ultraviolet light require that a person enter a room or area with the device. With such methods and devices, the person may introduce new contaminants to the room or area. Other methods and devices use disinfectants such as wipes, chemicals, and the like. However, airborne particles may settle on the surface treated with the wipes and/or chemicals. Moreover, traditional disinfection methods do not address the person introducing the contaminants to the room or area.

Implementations of the disclosed subject matter may deploy the mobile robot to a room, building, and/or area without putting a person (e.g., a member of a healthcare staff) at risk in a contaminated environment. That is, the mobile robot may disinfect air, surfaces, objects, and/or itself without putting a member of the healthcare staff at risk, may reduce the costs of protective equipment for a person, may reduce time in disinfecting, and/or may provide a report which includes details of the surfaces and/or objects that have been disinfected. Implementations of the disclosed subject matter may use UV light to disinfect the wheels and/or body of the mobile robot, so that it does not bring contaminants to other portions of a room or area, and/or to other locations.

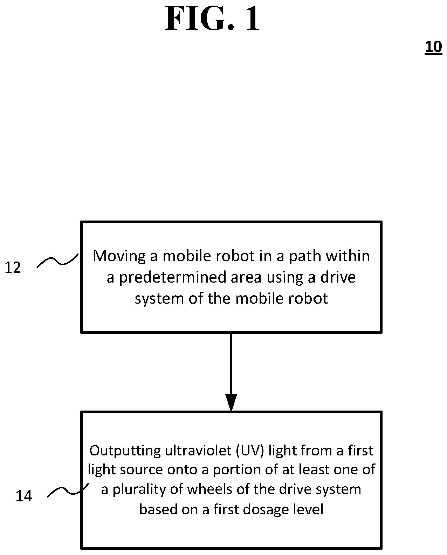

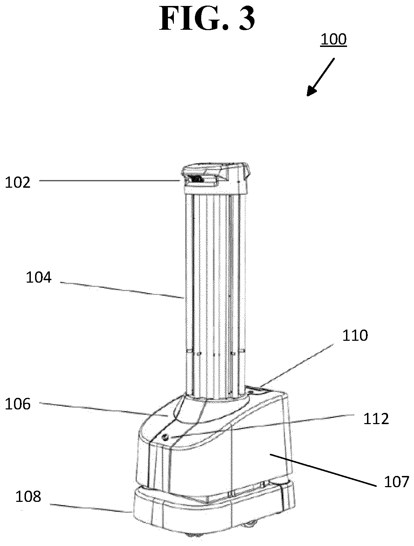

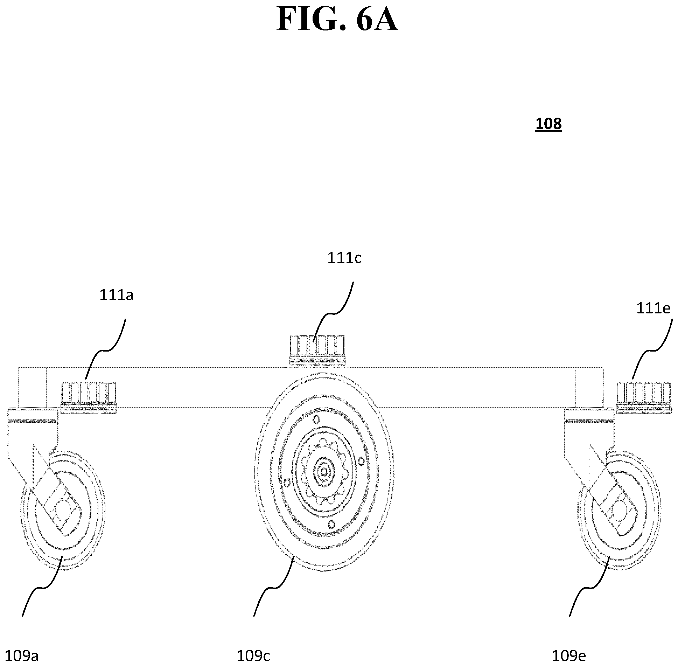

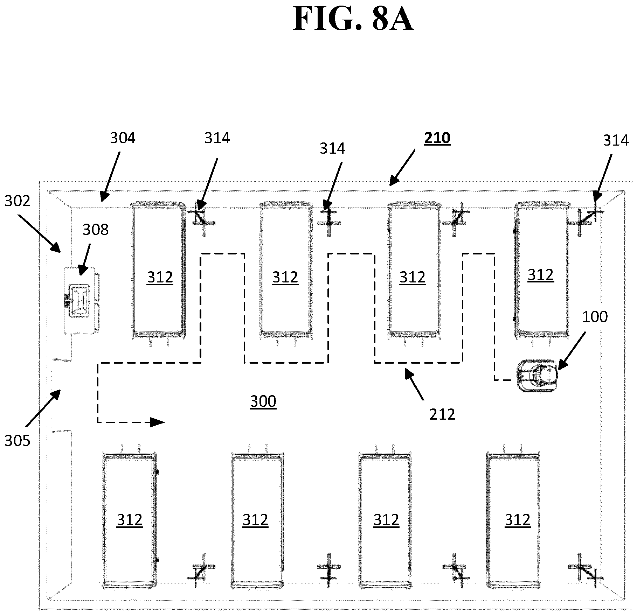

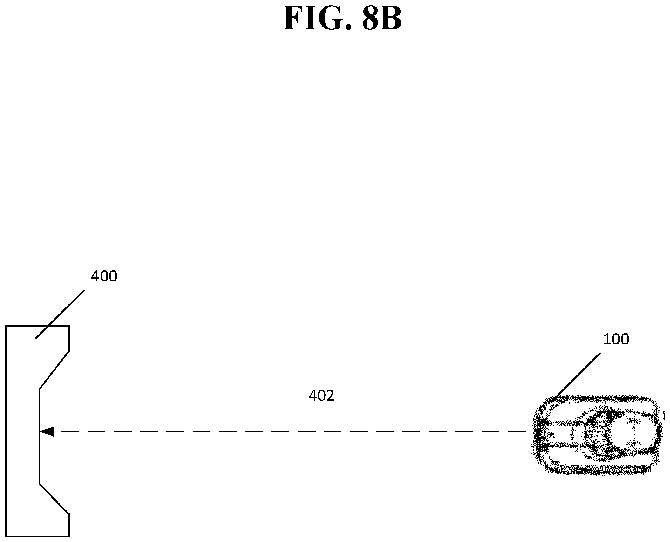

FIG. 1 shows a method 10 of operating a mobile robot (e.g., mobile robot 100 shown in FIGS. 3-11) to output ultraviolet (UV) according to an implementation of the disclosed subject matter. At operation 12, the mobile robot may be moved in a path (e.g., path 212 shown in FIG. 8A and/or path 402 shown in FIG. 8B) within a predetermined area using a drive system (e.g., drive system 108 shown in FIGS. 3, 6, and 10) of the mobile robot. At operation 14, UV light may be output from a first light source (e.g., light source 111a, 111b, 111c, 111d, 111e, 111f shown in FIGS. 6A-6D) onto a portion of at least one of a plurality of wheels (e.g., wheels 109a, 109b, 109c, 109d, 109e, 109f shown in FIGS. 6A-6D) of the drive system based on a first dosage level. The outputted UV light may disinfect one or more of the wheels of the mobile robot, which may prevent the spread of contaminants in one or more areas.

The UV light from the light source may be output onto at least a portion of a surface which the mobile robot is positioned on or moving over. For example, the mobile robot may be stationary on a floor in a building and may output light onto the floor over which the mobile robot is positioned. In another example, the UV light may be output from the first light source (e.g., light source 11 1a, 11 1b, 11 1c, 111d, 11 1e, 111f shown in FIGS. 6A-6D) when the mobile robot is moving over the floor or surface, such as when the mobile robot is moving along the path. In these examples, the UV light may disinfect the floor and/or other surface over which the mobile robot moves or is positioned over.

In some implementations, method 10 may include outputting UV light from a second light source (e.g., light source 104 shown in FIG. 3) onto one or more surfaces based on a second dosage level. The UV light output from the second light source when outputting the UV light from the first light source (e.g., light source 11 1a, 111b, 111c, 111d, 111e, 111f shown in FIGS. 6A-6D), and/or may output UV light from the second light source at a different time than outputting light from the first light source. The UV light output from the different light sources may disinfect surfaces, air, objects, and the like.

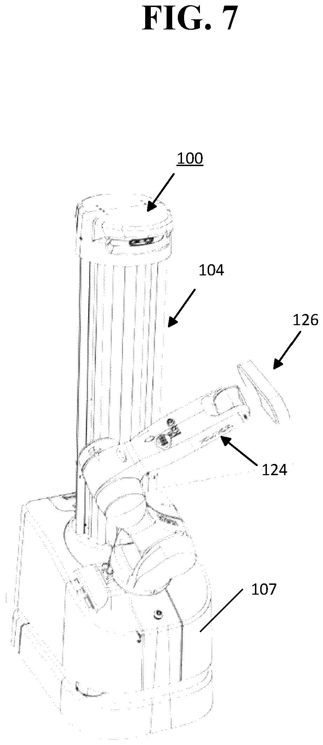

In some implementations, method 10 may include outputting light from a third light source (e.g., light source 126 shown in FIG. 7) onto at least a portion of a body (e.g., body 107 shown in FIGS. 3 and 7) of the mobile robot based on a third dosage level. That is, the first light source (e.g., light source 11 1a, 11 1b, 11 1c, 111d, 11 1e, 111f shown in FIGS. 6A-6D) may disinfect one or more of the wheels of the mobile robot, and the third light source (e.g., light source 126 shown in FIG. 7) may disinfect the body 107 of the mobile robot. The dosages of UV light output from the different light sources of the mobile robot may be different. The disinfection of the wheels and body of the mobile robot by one or more light sources may prevent the spread of contaminants to different areas.

The UV light may be output from the second light source (e.g., light source 104 shown in FIGS. 3 and 7) when outputting the UV light from the first light source (e.g., light source 126 shown in FIG. 7). In some implementations, the UV light may be output from the second light source at a different time than outputting light from the first light source. The UV light may be output from the third light source (e.g., light source 126 shown in FIG. 7) when outputting the UV light from the first light source. In some implementations, the UV light may be output from the third light source at a different time from when the UV light is output from the first light source and the second light source.

In the examples above, the dosage levels of UV light output from the different light sources may be different amplitudes of UV light, or may be the same dosage levels with the same amplitudes of UV light.

The first light source (e.g., light source 111a, 111b, 111c, 111d, 111e, 111f shown in FIGS. 6A-6D), second light source (e.g., light source 104 shown in FIGS. 3 and 7), and/or the third light source (e.g., light source 126 shown in FIG. 7) may output UV light from a light emitting diode (LED), and organic light emitting diode (OLED), a bulb, and/or a UV light source.

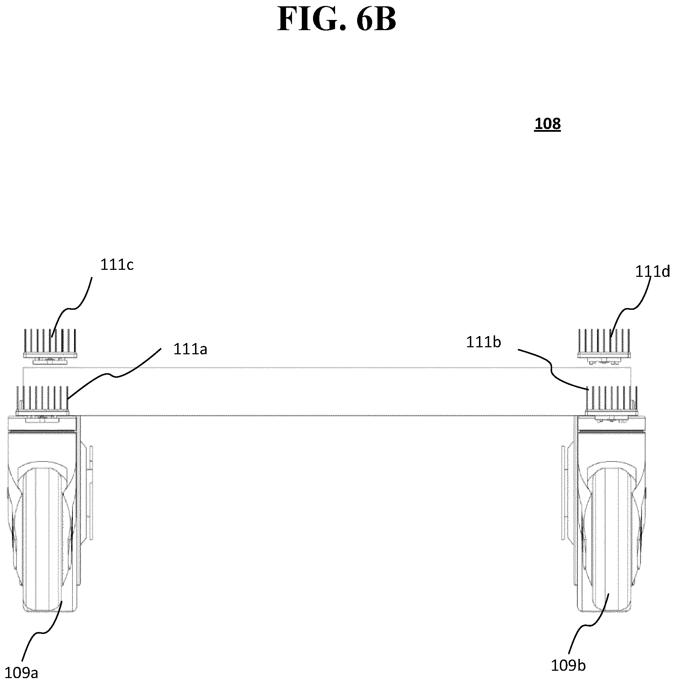

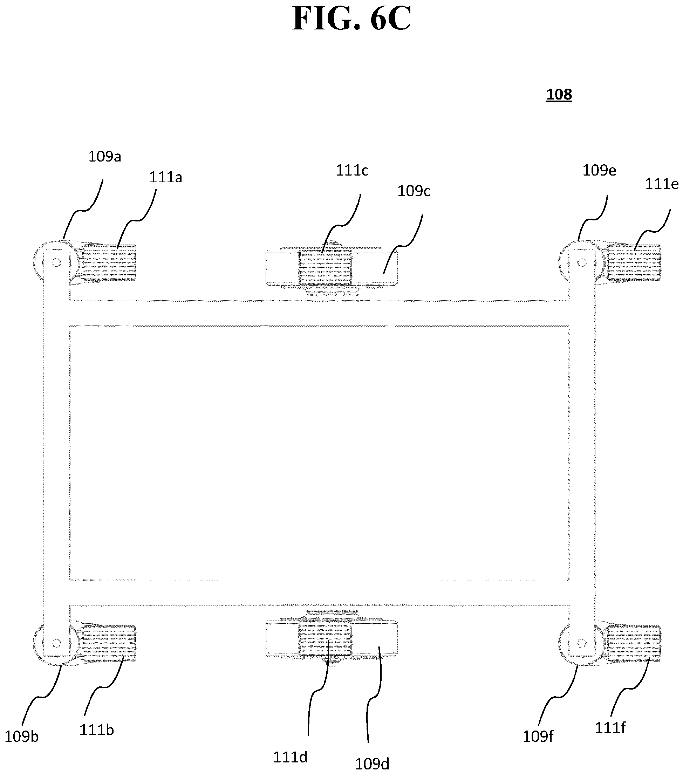

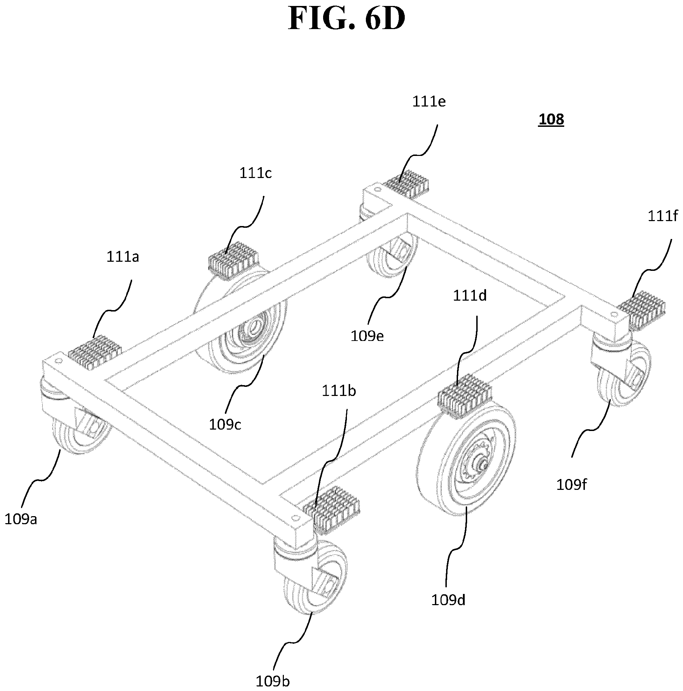

In some implementations, operation 14 of method 10 may include outputting the UV light from the first light source (e.g., light source 111a, 111b, 111c, 111d, 111e, 111f shown in FIGS. 6A-6D) onto the at least a portion of the wheels (e.g., wheels 109a, 109b, 109c, 109d, 109e, 109f, shown in FIG. 6) and the surface which the mobile robot is moving over when the mobile robot is moving towards a docking station (e.g., docking station 400 shown in FIG. 8B).

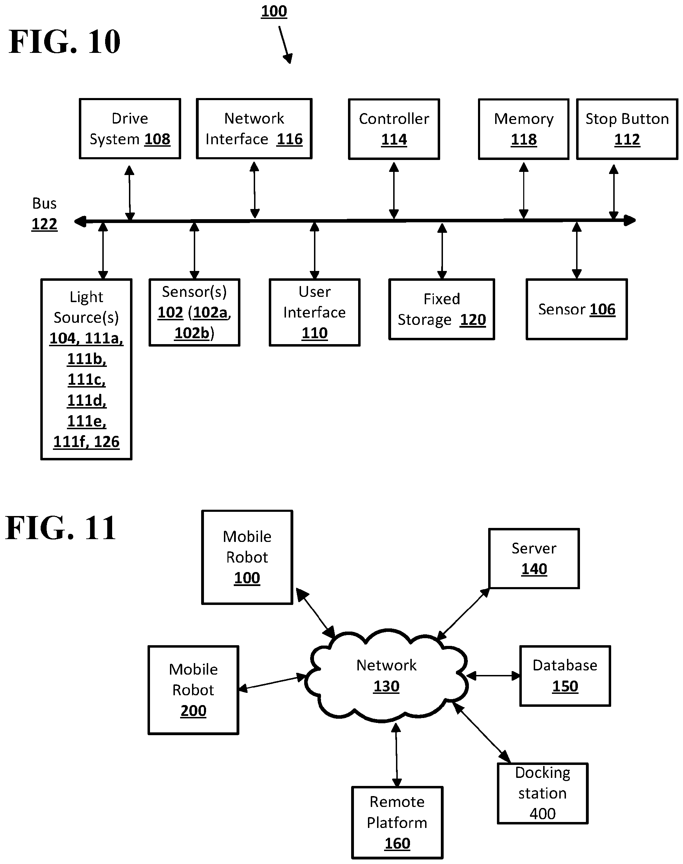

As shown in FIG. 8B, the mobile robot 100 may travel along path 402 to the docking station 400. The mobile robot 100 may recharge its battery (e.g., to power the drive system 108, the light sources 104, 111a, 111b, 111c, 111d, 111e, 111f, 126, and/or other components of the mobile robot shown in FIGS. 6A-6D and FIG. 10) at the docking station 400, and/or provide disinfection data (e.g., data regarding areas that the mobile robot disinfected) via a communications interface (e.g., network interface 116 shown in FIG. 10 or other communications interface). The mobile robot may receive instructions, paths, maps, or the like when coupled to the docking station 400. That is, the docking station may provide power to charge the battery of the mobile robot, and may receive data from and/or provide data to the mobile robot via a communications interface. The docking station 400 may be communicatively coupled to network 130 (e.g., as shown in FIG. 11), so that data may be provided from and/or transmitted to server 140, database 150, remote platform 160, and/or another mobile robot (e.g., mobile robot 200).

In some implementations, the mobile robot 100 may disinfect portions of the wheels 109a, 109b, 109c, 109d, 109e, 109f and/or the body 107 as the mobile robot moves between a first area and a second area. The first area and/or the second area may be different rooms, different portions of a room, a hallway and a room, different portions of a predetermined area, or the like.

In some implementations, method 10 may include adjusting, by a controller of the mobile robot (e.g., controller 114 shown in FIG. 10), an orientation of the first light source (e.g., light source 111a, 111b, 111c, 111d, 111e, 111f shown in FIGS. 6A-6D) so that the first light source outputs UV light onto at least a portion of one of the plurality of wheels (e.g., wheels 109a, 109b, 109c, 109d, 109e, 109f shown in FIGS. 6A-6D). That is, the light source 111a, 111b, 111c, 111d, 111e, and/or 111f may be mounted so as to be actuated to change their orientation about one or more axes. This may change the direction that UV light may be output from the light source 111a, 111b, 111c, 111d, 111e, 111f The mounts for light source 111a, 111b, 111c, 111d, 111e, and/or 111f may include multi-axial mechanical mounts that may be oriented with one or more electrical motors (e.g., that are controlled by the controller of the mobile robot).

In some implementations, the controller communicatively coupled to the mobile robot may determine the path (e.g., path 212 shown in FIG. 8A and/or path 402 shown in FIG. 8B) of the mobile robot. For example, as shown in FIG. 8A, the controller may determine the path 212 for the mobile robot 100 in room 210, having walls (e.g., surfaces 302, 304), floor (e.g., surface 300), object 308 (e.g., a sink), object 312 (e.g., a bed), object 314 (e.g., 4-hook IV stand), and the like. The path may be determined so that the mobile robot may output a dosage of UV light to the objects 308, 312, 314, and the surfaces 300, 302, 304 to disinfect them in, for example, the shortest amount of time. The controller may be, for example, controller 114 shown in FIG. 10, and/or server 140 and/or remote platform 160 which may be communicatively coupled to the mobile robot 100 via the network 130 as shown in FIG. 11. In some implementations, the determined path may be a random path.

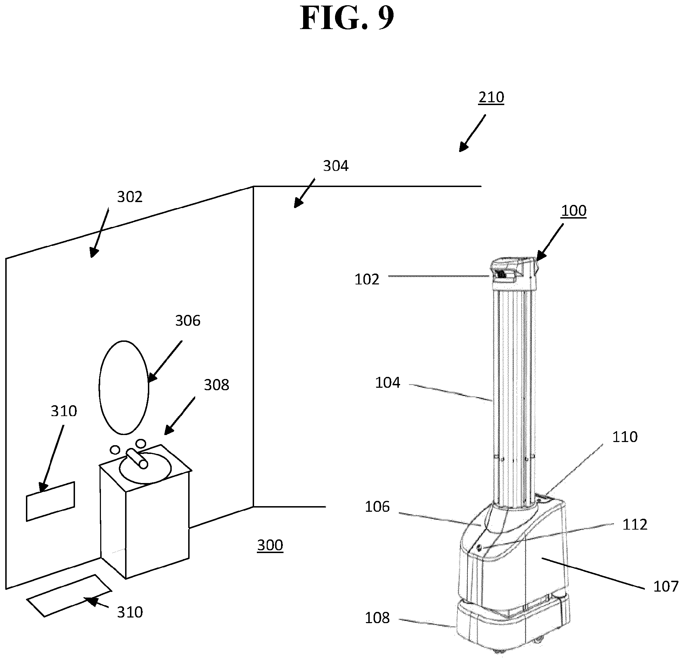

In some implementations, the path (e.g., path 212 shown in FIG. 8A and/or path 402 shown in FIG. 8B) may be determined based on an environment of the predetermined area (e.g., room 210 shown in FIGS. 8A and 9), providing a reduced time for disinfection of the predetermined area, and/or increasing the dosage to the one or more surfaces (e.g., surfaces 300, 302, 304 shown in FIGS. 8A and 99; objects 306, 308, 312, 314 shown in FIGS. 8A and 9; and/or reference tag 310 shown in FIG. 9).

The path may be determined, for example, based at least in part on a two dimensional map or a three-dimensional map generated by the controller (e.g., controller 114 shown in FIG. 10, and/or server 140 and/or remote platform 160 which may be communicatively coupled to the mobile robot 100 via the network 130 as shown in FIG. 14) and at least one sensor (e.g., sensor 102 and/or sensor 106) of the mobile robot moving within the predetermined area (e.g., room 210) at a previous point in time.

In some implementations, the path may be determined based on an amount of UV light that is to be output from the light source (e.g., light source 104, 1 1 1a, 111b, 111c, 111d, 111e, 111f, 126 shown in FIGS. 3, 6A-6D, and 7) on the one or more surfaces (e.g., surfaces 300, 302, 304 shown in FIGS. 8A and 9; objects 306, 308, 312, 314 shown in FIGS. 8A and 9; and/or reference tag 310 shown in FIG. 9). In some implementations, the path may be a perimeter of the predetermined area (e.g., a perimeter of room 210 shown in FIG. 8A), and/or a path to the docking station (e.g., path 402 shown in FIG. 8B). In some implementations, the moving the mobile within the path may include moving the mobile robot in a predetermined pattern (e.g., a grid pattern along a determined pattern within the predetermined area, or the like).

In some implementations, the method 10 may include using the controller communicatively coupled to the mobile robot (e.g., controller 114 shown in FIG. 10, and/or server 140 and/or remote platform 160 which may be communicatively coupled to the mobile robot 100 via the network 130 as shown in FIG. 11) to determine whether the one or more surfaces (e.g., surfaces 300, 302, 304 shown in FIGS. 8A and 9; objects 306, 308, 312, 314 shown in FIGS. 8A and 9; and/or reference tag 310 shown in FIG. 9) have received a dosage of UV light.

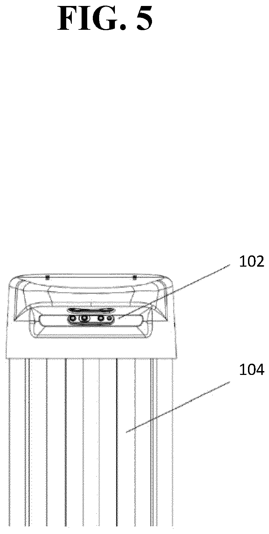

In some implementations, the mobile robot may use a sensor (e.g., sensor 102, 106 shown in FIG. 5) to detect at least one hotspot within the predetermined area (e.g., room 210). The hotspot may be a predetermined object (e.g., objects 306, 308, 312, 314 shown in FIGS. 8A and 9; and/or reference tag 310 shown in FIG. 9), at least a portion of the predetermined area (e.g., surfaces 300, 302, 304 shown in FIGS. 8A and 9), an object having a predetermined type of contaminant, and/or a portion of the floor and/or surface over which the mobile robot may travel (e.g., surface 300 and/or path 212 shown in FIG. 8A, and/or path 402 shown in FIG. 8B). For example, the at least one hotspot may be a chair, a seat, a bed, a sink, mirror, a door, a door handle, a wall, a floor, a ceiling, a shelf, a surface of a table, and any object and/or surface defined as the at least one hotspot in a memory (e.g., memory 118 and/or fixed storage 120 shown in FIG. 10; database 150 shown in FIG. 11) that is communicatively coupled to a controller (e.g., controller 114 shown in FIG. 10, and/or server 140 and/or remote platform 160 which may be communicatively coupled to the mobile robot 100 via the network 130 as shown in FIG. 11) of the mobile robot.

In some implementations, the UV light may be output from the at least one light source (e.g., light source 104 shown in FIG. 3, light source 111a, 111b, 111c, 111d, 111e, 111f shown in FIGS. 6A-6D, and/or light source 126 shown in FIG. 7) at a second dosage onto the at least one hotspot. This second dosage may be greater than the first dosage. That is, the intensity and/or duration of the UV light output to the hotspot based on the second dosage may be greater that the intensity and/or duration of the UV light output based on the first dosage.

In some implementations, the mobile robot may transmit, from a communications interface (e.g., network interface 116), data including the one or more surfaces (e.g., surfaces 300, 302, 304 shown in FIGS. 8A and 9; objects 306, 308, 312, 314 shown in FIGS. 8A and 9; and/or reference tag 310 shown in FIG. 9) that have received a dosage of UV light.

FIG. 7 shows that the mobile robot 100 may include an adjustable, where the arm includes another light source (e.g., light source 126) to output UV light, such as onto the body 107 of the mobile robot 100 according to an implementation of the disclosed subject matter. The controller (e.g., controller 114 shown in FIG. 10) of the mobile robot 100 may adjust the arm 124, and may control the operation of the light source 126. The light source 126 may output UV light onto, for example, the body 107 of the mobile robot 100, surfaces 300, 302, 304 shown in FIGS. 8A and 9, objects 306, 308, 312, 314 shown in FIGS. 8A and 9, and/or reference tag 310 shown in FIG. 9. The controller may determine the dosage level of UV light to be output, depending upon the surface and/or object to be radiated, including the body 107 of the mobile robot.

In some implementations, the mobile robot 100 may detect air, surfaces, and/or objects of an area to disinfect them with the UV light as shown in FIG. 9. For example, sensors 102 and/or 106 of the mobile robot 100 may be used to detect surface 300 (e.g., a floor of the area), surface 302 and/or surface 306 (e.g., a wall of the area). The sensors 102 and/or 106 may be used to detect object 306 (e.g., a mirror), object 308 (e.g., a sink), and/or reference tag 310. The reference tag 310 may have a first state, and may change to a second state when a dosage of UV light is applied to the reference tag 310. In some implementations, the reference tag 310 may be a virtual reference tag that is represented in a map of the area, which may changes states when UV light is applied to the area that corresponds with the mapped area. In some implementations, the controller (e.g., controller 114 shown in FIG. 10) may determine that one or more of the objects 306, 308 are hotspots. UV light may be emitted by the light source 104 to disinfect the surfaces 300, 302, 304 and/or the objects 306, 308. The map and the exposure plot may be generated by the controller of the mobile robot 100.

FIGS. 3-6D show a plurality of external views of the mobile robot 100 that includes sensors to detect surfaces and objects in an area, and a light source to output UV light having a first dosage based on a received dosage level to disinfect the air, objects, and/or surfaces in the area according to implementations of the disclosed subject matter. The mobile robot 100 having body 107 may include at least a first sensor 102 (shown as sensor 102a and 102b in FIG. 3), a light source 104 to output ultraviolet light, at least a second sensor 106, a drive system 108, a user interface 110, and/or a stop button 112. A controller (e.g., controller 114 shown in FIG. 10 and described below) may be communicatively coupled to the at least one first sensor 102, the light source 104, the at least one second sensor 106, the drive system 108, the user interface 110 and the stop button 112, may control the operations of the mobile robot 100.

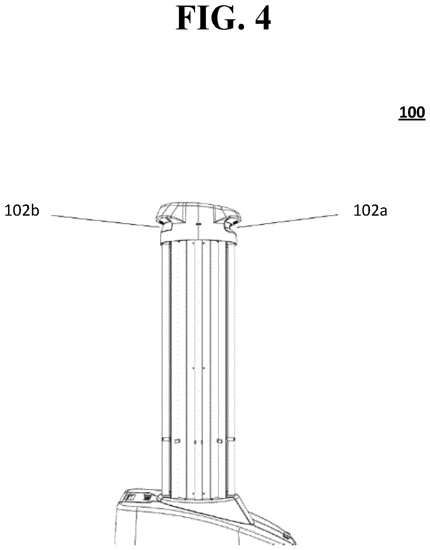

The at least one first sensor 102 (including sensors 102a, 102b shown in FIG. 4) may determine at least one of an orientation of the mobile robot 100 (e.g., a direction that a front side and/or a first side of a robot is facing), a location of the mobile robot 100 (e.g., a location of the mobile robot 100 in an area), and/or when the light source 104 is within a predetermined distance of a surface and/or object in the area (e.g., surface 300, 302, and/or 304, and/or object 306, 308 shown in FIGS. 8A and 9). In some implementations, the first sensor 102 may detect air, a surface, a reference tag, and/or objects that may disinfected with UV light from the light source 104.

In some implementations, the at least one first sensor 102 may have a field of view of 70 degrees diagonally. The at least one sensor 102 may have a detection distance of 0.2 - 4 meters. As shown in FIGS. 3-5, the at least one first sensor 102 may be disposed over the light source 104.

The at least one first sensor 102 may include a first side sensor disposed on a first side of the mobile robot 100 and a second side sensor that may be disposed on a second side of the device. For example, as shown in FIG. 4, sensor 102a may be disposed on a first side (e.g., a front side) of the mobile robot 100, and sensor 102b may be disposed on a second side (e.g., a back side) of the mobile robot 100. Although sensors on two sides of the robot are shown in FIG. 4, there may be a plurality of sensors disposed on different sides of the mobile robot 102 to at least detect surfaces and/or objects. In some implementations, sensor 102a and/or sensor 102b may be disposed over the light source 104.

The light source 104 may be one or more bulbs, one or more lamps, and/or an array of light emitting diodes (LEDs) or organic light emitting diodes (OLEDs) to emit UV light (e.g., light having a wavelength of 10 nm - 400 nm). The dosage of the UV light (e.g., intensity, duration, optical power output, or the like) may be controlled by the controller 114, which may also turn on or off a portion or all of the devices (e.g., bulbs, lamps, LEDs, OLEDs) of the light source 104. The light source may be controlled to emit UV light when the mobile robot is within an area, as the mobile robot moves within the area, before the mapping of the area, during the mapping of the area, and/or after the mapping of the area.

In some implementations, the mobile robot may include light source 126 which may be coupled to a robotic arm 124 of the mobile robot 100. The light source 126 may emit UV light from one or more bulbs, one or more lamps, and/or an array of light emitting diodes (LEDs) or organic light emitting diodes (OLEDs) to emit UV light (e.g., light having a wavelength of 10 nm - 400 nm). The light source 126 may be controlled to emit UV light. In some implementations, the light source 126 may be used to provide a dosage of UV light to the body 107 of the mobile robot 100, air, objects, surfaces, reference tags, or the like. Movement of the arm 124 may be controlled by the controller 114 shown in FIG. 10.

The at least one second sensor 106 may be communicatively coupled to the controller 114 shown in FIG. 10, and may be used to detect air, surfaces, and/or objects that may be disinfected with UV light from the light source 104. In some implementations, the at least one second sensor 106 may determine at least one of an orientation of the mobile robot 100 (e.g., a direction that a front side and/or a first side of a robot is facing), a location of the mobile robot 100 (e.g., a location of the mobile robot 100 in an area), and/or when the light source 104 is within a predetermined distance of a surface and/or object in the area (e.g., surface 300, 302, and/or 304, and/or object 306, 308 shown in FIG. 9).

In some implementations, the sensor 102, 106 may be a time-of-flight sensor, an ultrasonic sensor, a two-dimensional (2D) Light Detection and Ranging (LiDAR) sensor, a three-dimensional (3D) LiDAR sensor, and/or a radar (radio detection and ranging) sensor, a stereo vision sensor, 3D three camera, a structured light camera, or the like. The sensor 106 may have a field of view of 20-27 degrees. In some implementations, the sensor 106 may have a detection distance of 0.05 - 4 meters.

The mobile robot 100 may include a motor to drive the drive system 108 to move the mobile robot in an area, such as a room, a building, or the like. The drive system 108 may be part of body 107, and the sensor 102, 106 may be disposed on the body 107. The drive system 108 may include wheels, which may be adjustable so that the drive system 108 may control the direction of the mobile robot 100.

FIGS. 6A-6D show a bottom view of the drive system 108, which includes wheels 109a, 109b, 109c, 109d, 109e, and/or 109f, and light source 111a, 111b, 111c, 111d, 111e, and/or 111f which may output UV light at a dosage controlled by the controller 114 shown in FIG. 10. The light source 111a, 111b, 111c, 111d, 111e, 111f may be one or more bulbs, one or more lamps, and/or an array of light emitting diodes (LEDs) or organic light emitting diodes (OLEDs) to emit UV light (e.g., light having a wavelength of 10 nm - 400 nm). The UV light may be emitted onto at least a portion of one or more of the wheels 109a, 109b, 109c, 109d, 109e, 109f and/or onto a surface over which the drive system 108 is positioned on and/or moving over.

The controller 114 may control and/or operate the mobile robot 100 in an operation mode which may be a manual mode, an autonomous mode, and/or a tele-operation mode. In the manual mode, the controller 114 may receive on or more control signals from the user interface 110 and/or the stop button 112. For example, a user may control the movement, direction, and/or stop the motion of the mobile robot 100 by making one or more selections on the user interface 110. The stop button 112 may be an emergency stop (ESTOP) button which may stop all operations and/or movement of the mobile robot 100 when selected. In some implementations, the controller 114 may receive at least one control signal via a network interface 116 (shown in FIG. 10) when operating when operating in the tele-operation mode. For example, the network interface may receive control signals via network 130 from server 140, database 150, and/or remote platform 160, as described below in connection with FIG. 11.

In some implementations, when the mobile robot 100 is moving in a direction, the sensor 102, 106 may detect a geometry of one or more surfaces (e.g., surfaces 300, 302, 304 shown in FIG. 9), objects (e.g., objects 306, 308 shown in FIG. 9), and/or reference tags (e.g., reference tag 310 shown in FIG. 9). The output of the at least one first sensor 102 may be, for example, a point cloud of the one or more objects in the path of the mobile robot 100. When the sensor 102 and/or sensor 106 is a stereo vision sensor, images from two sensors (i.e., where the two sensors may be part of the stereo vision sensor of the sensor 102 and/or sensor 106) within a known distance from one another distance may be captured at a predetermined point in time, and/or at predetermined time intervals with a global shutter. The global shutter may be configured so that the two sensors of the stereo vision sensor may capture images about simultaneously. One or more features (e.g., surfaces 300, 302, 304, and/or objects 306, 308, and/or reference tag 310 shown in FIG. 9) may be determined from the captured images, and be compared to one another to determine portions that are matching. As the focal length of the two sensors of the stereo vision sensor and the distance between the two sensors (e.g., about 6 cm) may be stored in memory 118 and/or fixed storage 120 (shown in FIG. 10), the controller 114 and/or the at least one first sensor 102 may use the captured images and the stored values to determine the distance from the sensor 102, 106 to the surfaces and/or objects, and may be used by the controller for outputting a dosage of UV light from the light source. In some implementations, the sensor 102, 106 may include at least one laser, LED, and/or OLED, to radiate one or more points on surfaces of objects, when the objects may be without identifying features (e.g., blank walls).

When detecting the surface and/or object, the sensor 102, 106 may be a time-of-flight (TOF) sensor. At least one photon of light may be output by the sensor 102, 106, and may be transmitted through the air. When the at least one photon of light radiates surface and/or an object, a portion of the light may be reflected by the surface and/or the object may return to a receiver portion of the sensor 102, 106. The sensor 106 may calculate the time between sending the at least one photon of light and receiving the reflection, and multiply this value by the speed of light in air, to determine the distance between the sensor 102, 106 and surface and/or object. This may be used to generate the map of the area that the mobile robot is operating within.

FIG. 10 shows example components of the mobile robot 100 suitable for providing the implementations of the disclosed subject matter. The mobile robot 100 may include a bus 122 which interconnects major components of the mobile robot 100, such as the drive system 108, a network interface 116 operable to communicate with one or more remote devices via a suitable network connection, the controller 114, a memory 118 such as Random Access Memory (RAM), Read Only Memory (ROM), flash RAM, or the like, the stop button 112, the light source 104, 111a, 111b, 111c, 111d, 111e, 111f and/or 126, the at least one first sensor 102, a user interface 110 that may include one or more controllers and associated user input devices such as a keyboard, touch screen, and the like, a fixed storage 120 such as a hard drive, flash storage, and the like, and the at least one second sensor 106.

The bus 122 allows data communication between the controller 114 and one or more memory components, which may include RAM, ROM, and other memory, as previously noted. Typically RAM is the main memory into which an operating system and application programs are loaded. A ROM or flash memory component can contain, among other code, the Basic Input-Output system (BIOS) which controls basic hardware operation such as the interaction with peripheral components. Applications resident with the mobile robot 100 are generally stored on and accessed via a computer readable medium (e.g., fixed storage 120), such as a solid state drive, hard disk drive, an optical drive, solid state drive, or other storage medium.

The network interface 116 may provide a direct connection to a remote server (e.g., server 140, database 150, and/or remote platform 160 shown in FIG. 10) via a wired or wireless connection (e.g., network 130 shown in FIG. 10). The network interface 116 may provide such connection using any suitable technique and protocol as will be readily understood by one of skill in the art, including digital cellular telephone, WiFi, Bluetooth(R), near-field, and the like. For example, the network interface 116 may allow the mobile robot 100 to communicate with other computers via one or more local, wide-area, or other communication networks, as described in further detail below. The mobile robot may transmit data via the network interface to the remote server that may include a path of operation, the surfaces and/or areas radiated with UV light, and the like.

Many other devices or components (not shown) may be connected in a similar manner. Conversely, all of the components shown in FIG. 10 need not be present to practice the present disclosure. The components can be interconnected in different ways from that shown. Code to implement the present disclosure can be stored in computer-readable storage media such as one or more of the memory 118, fixed storage 120, or on a remote storage location.

FIG. 11 shows an example network arrangement according to an implementation of the disclosed subject matter. Mobile robot 100 described above, and/or a similar mobile robot 200 may connect to other devices via network 130. The network 130 may be a local network, wide-area network, the Internet, or any other suitable communication network or networks, and may be implemented on any suitable platform including wired and/or wireless networks. The mobile robot 100 and/or mobile robot 200 may communicate with one another, and/or may communicate with one or more remote devices, such as server 140, database 150, and/or remote platform 160. The remote devices may be directly accessible by the mobile robot 100, 200 or one or more other devices may provide intermediary access such as where a server 140 provides access to resources stored in a database 150. The mobile robot 100, 200 may access remote platform 160 or services provided by remote platform 160 such as cloud computing arrangements and services. The remote platform 160 may include one or more servers 140 and/or databases 150. In some implementations, remote platform 160 and/or server 140 may remotely control the mobile robot 100 and/or mobile robot 200.

More generally, various implementations of the presently disclosed subject matter may include or be embodied in the form of computer-implemented processes and apparatuses for practicing those processes. Implementations also may be embodied in the form of a computer program product having computer program code containing instructions embodied in non-transitory and/or tangible media, such as solid state drives, DVDs, CD-ROMs, hard drives, USB (universal serial bus) drives, or any other machine readable storage medium, such that when the computer program code is loaded into and executed by a computer, the computer becomes an apparatus for practicing implementations of the disclosed subject matter. Implementations also may be embodied in the form of computer program code, for example, whether stored in a storage medium, loaded into and/or executed by a computer, or transmitted over some transmission medium, such as over electrical wiring or cabling, through fiber optics, or via electromagnetic radiation, such that when the computer program code is loaded into and executed by a computer, the computer becomes an apparatus for practicing implementations of the disclosed subject matter. When implemented on a general-purpose microprocessor, the computer program code segments configure the microprocessor to create specific logic circuits.

In some configurations, a set of computer-readable instructions stored on a computer-readable storage medium may be implemented by a general-purpose processor, which may transform the general-purpose processor or a device containing the general-purpose processor into a special-purpose device configured to implement or carry out the instructions. Implementations may include using hardware that has a processor, such as a general purpose microprocessor and/or an Application Specific Integrated Circuit (ASIC) that embodies all or part of the techniques according to implementations of the disclosed subject matter in hardware and/or firmware. The processor (e.g., controller 114 shown in FIG. 10) may be coupled to memory, such as RAM, ROM, flash memory, a hard disk or any other device capable of storing electronic information. The memory may store instructions adapted to be executed by the processor to perform the techniques according to implementations of the disclosed subject matter.

The foregoing description, for purpose of explanation, has been described with reference to specific implementations. However, the illustrative discussions above are not intended to be exhaustive or to limit implementations of the disclosed subject matter to the precise forms disclosed. Many modifications and variations are possible in view of the above teachings as long as they fall within the scope of the claims.

The implementations were chosen and described in order to explain the principles of implementations of the disclosed subject matter and their practical applications, to thereby enable others skilled in the art to utilize those implementations as well as various implementations with various modifications as may be suited to the particular use contemplated.

## Claims

1. A method (10) comprising:
moving (12) a mobile robot (100) in a path within a predetermined area using a drive system of the mobile robot (100); and
outputting (14) ultraviolet (UV) light from a first light source onto a portion of at least one of a plurality of wheels of the drive system based on a first dosage level,
**characterized by** outputting the UV light from the first light source onto at least a portion of a surface which the mobile robot (100) is positioned on or moving over.

2. The method (10) of any preceding claim, further comprising:
outputting UV light from a second light source onto one or more surfaces based on a second dosage level.

3. The method (10) of claim 2, wherein the outputting the UV light comprises at least one selected from the group consisting of: outputting UV light from the second light source when outputting the UV light from the first light source; and outputting UV light from the second light source at a different time than outputting light from the first light source.

4. The method (10) of any one of claims 2 to 3, further comprising:
outputting light from a third light source onto at least a portion of a body of the mobile robot (100) based on a third dosage level.

5. The method (10) of claim 4, wherein the outputting the UV light comprises at least one selected from the group consisting of: outputting the UV light from the second light source when outputting the UV light from the first light source; outputting the UV light from the second light source at a different time than outputting light from the first light source; outputting the UV light from the third light source when outputting the UV light from the first light source; and outputting the UV light from the third light source at a different time from outputting the UV light from the first light source and the second light source.

6. The method (10) of any one of claims 2 to 5, wherein the first dosage level and the second dosage level output different amplitudes of UV light.

7. The method (10) of claim 6, wherein the amplitude of the second dosage level is less than the amplitude of the first dosage level.

8. The method (10) of any one of claims 2 to 7, wherein at least one of the first light source and the second light source outputs UV light from at least one selected from the group consisting of: a light emitting diode (LED), and organic light emitting diode (OLED), a bulb, and a UV light source.

9. The method (10) of any preceding claim, wherein the outputting UV light from the first light source further comprises:
outputting the UV light when the mobile robot (100) is moving in the path or when the robot is stationary.

10. The method (10) of any preceding claim, wherein the outputting the UV from the first light source further comprises:
outputting the UV light from the first light source onto the at least a portion of the wheels and the surface which the mobile robot (100) is moving over when the mobile robot (100) is moving towards a docking station or moving between a first area and a second area.

11. The method (10) of any preceding claim, wherein the outputting UV light from the first light source comprises:
adjusting, by a controller of the mobile robot (100), an orientation of the first light source so that the first light source outputs UV light onto at least a portion of one of the plurality of wheels.

## Patentansprüche

1. Verfahren (10), umfassend:
Bewegen (12) eines mobilen Roboters (100) auf einer Bahn innerhalb eines vorher bestimmten Bereichs unter Verwendung eines Antriebssystems des mobilen Roboters (100); und
Ausgeben (14) von ultraviolettem Licht (UV-Licht) von einer ersten Lichtquelle auf einen Abschnitt von mindestens einem von mehreren Rädern des Antriebssystems basierend auf einer ersten Dosierung,
**gekennzeichnet durch** das Ausgeben des UV-Lichts von der ersten Lichtquelle auf mindestens einen Abschnitt einer Fläche, auf der der mobile Roboter (100) positioniert ist oder sich gerade bewegt.

2. Verfahren (10) nach einem der vorhergehenden Ansprüche, ferner umfassend:
Ausgeben von UV-Licht von einer zweiten Lichtquelle auf eine oder mehrere Flächen basierend auf einer zweiten Dosierung.

3. Verfahren (10) nach Anspruch 2, wobei das Ausgeben des UV-Lichts mindestens einen Vorgang umfasst, der aus der Gruppe ausgewählt ist, die aus Folgendem besteht: Ausgeben von UV-Licht von der zweiten Lichtquelle beim Ausgeben des UV-Lichts von der ersten Lichtquelle; und Ausgeben von UV-Licht von der zweiten Lichtquelle zu einer anderen Zeit als beim Ausgeben von Licht von der ersten Lichtquelle.

4. Verfahren (10) nach einem der Ansprüche 2 bis 3, ferner umfassend:
Ausgeben von Licht von einer dritten Lichtquelle auf mindestens einen Abschnitt eines Körpers des mobilen Roboters (100) basierend auf einer dritten Dosierung.

5. Verfahren (10) nach Anspruch 4, wobei das Ausgeben des UV-Lichts mindestens einen Vorgang umfasst, der aus der Gruppe ausgewählt ist, die aus Folgendem besteht: Ausgeben des UV-Lichts von der zweiten Lichtquelle beim Ausgeben des UV-Lichts von der ersten Lichtquelle; Ausgeben des UV-Lichts von der zweiten Lichtquelle zu einer anderen Zeit als beim Ausgeben von Licht von der ersten Lichtquelle; Ausgeben des UV-Lichts von der dritten Lichtquelle beim Ausgeben des UV-Lichts von der ersten Lichtquelle; und Ausgeben des UV-Lichts von der dritten Lichtquelle zu einer anderen Zeit als beim Ausgeben des UV-Lichts von der ersten Lichtquelle und der zweiten Lichtquelle.

6. Verfahren (10) nach einem der Ansprüche 2 bis 5, wobei durch die erste Dosierung und die zweite Dosierung UV-Licht mit unterschiedlichen Amplituden ausgegeben wird.

7. Verfahren (10) nach Anspruch 6, wobei die Amplitude der zweiten Dosierung kleiner als die Amplitude der ersten Dosierung ist.

8. Verfahren (10) nach einem der Ansprüche 2 bis 7, wobei mindestens eine von der ersten Lichtquelle und der zweiten Lichtquelle UV-Licht von mindestens einem Element ausgibt, das aus der Gruppe ausgewählt ist, die aus Folgendem besteht: einer Leuchtdiode (LED) und einer organischen Leuchtdiode (OLED), einer Glühlampe und einer UV-Lichtquelle.

9. Verfahren (10) nach einem der vorhergehenden Ansprüche, wobei das Ausgeben von UV-Licht von der ersten Lichtquelle ferner Folgendes umfasst:
Ausgeben des UV-Lichts, während sich der mobile Roboter (100) auf der Bahn bewegt oder wenn der Roboter stationär ist.

10. Verfahren (10) nach einem der vorhergehenden Ansprüche, wobei das Ausgeben des UV-Lichts von der ersten Lichtquelle ferner Folgendes umfasst:
Ausgeben des UV-Lichts von der ersten Lichtquelle auf den mindestens einen Abschnitt der Räder und der Fläche, auf der sich der mobile Roboter (100) gerade bewegt, während sich der mobile Roboter (100) zu einer Dockingstation hin bewegt oder sich zwischen einem ersten Bereich und einem zweiten Bereich bewegt.

11. Verfahren (10) nach einem der vorhergehenden Ansprüche, wobei das Ausgeben von UV-Licht von der ersten Lichtquelle Folgendes umfasst:
Einstellen, durch eine Steuerung des mobilen Roboters (100), einer Ausrichtung der ersten Lichtquelle, sodass die erste Lichtquelle UV-Licht auf mindestens einen Abschnitt eines der mehreren Räder ausgibt.

## Revendications

1. Procédé (10) comprenant :
un déplacement (12) d'un robot mobile (100) dans une trajectoire au sein d'une zone prédéterminée à l'aide d'un système d'entraînement du robot mobile (100) ; et
une délivrance (14) de lumière ultraviolette (UV) à partir d'une première source de lumière sur une partie d'au moins une parmi une pluralité de roues du système d'entraînement sur la base d'un premier niveau de dosage,
**caractérisé par** une délivrance de la lumière UV à partir de la première source de lumière sur au moins une partie d'une surface sur laquelle le robot mobile (100) est positionné ou se déplace.

2. Procédé (10) selon une quelconque revendication précédente, comprenant en outre :
une délivrance de lumière UV à partir d'une deuxième source de lumière sur une ou plusieurs surfaces sur la base d'un deuxième niveau de dosage.

3. Procédé (10) selon la revendication 2, dans lequel la délivrance de la lumière UV comprend au moins un élément choisi parmi le groupe composé : d'une délivrance de lumière UV à partir de la deuxième source de lumière lors d'une délivrance de la lumière UV à partir de la première source de lumière ; et d'une délivrance d'une lumière UV à partir de la deuxième source de lumière à un instant différent de la délivrance de lumière à partir de la première source de lumière.

4. Procédé (10) selon une quelconque des revendications 2 à 3, comprenant en outre :
une délivrance de lumière à partir d'une troisième source de lumière sur au moins une partie d'un corps du robot mobile (100) sur la base d'un troisième niveau de dosage.

5. Procédé (10) selon la revendication 4, dans lequel la délivrance de la lumière UV comprend au moins un élément choisi parmi le groupe composé : d'une délivrance de lumière UV à partir de la deuxième source de lumière lors d'une délivrance de la lumière UV à partir de la première source de lumière ; d'une délivrance de la lumière UV à partir de la deuxième source de lumière à un instant différent d'une délivrance de lumière à partir de la première source de lumière ; d'une délivrance de la lumière UV à partir de la troisième source de lumière lors d'une délivrance de la lumière UV à partir de la première source de lumière ; et d'une délivrance de la lumière UV à partir de la troisième source de lumière à un instant différent d'une délivrance de la lumière UV à partir de la première source de lumière et de la deuxième source de lumière.

6. Procédé (10) selon une quelconque des revendications 2 à 5, dans lequel le premier niveau de dosage et le deuxième niveau de dosage délivrent des amplitudes différentes de lumière UV.

7. Procédé (10) selon la revendication 6, dans lequel l'amplitude du deuxième niveau de dosage est inférieure à l'amplitude du premier niveau de dosage.

8. Procédé (10) selon une quelconque des revendications 2 à 7, dans lequel au moins une parmi la première source de lumière et la deuxième source de lumière délivre une lumière UV à partir d'au moins un élément choisi parmi le groupe composé : d'une diode électroluminescente (LED), et d'une diode électroluminescente organique (OLED), d'une ampoule, et d'une source de lumière UV.

9. Procédé (10) selon une quelconque revendication précédente, dans lequel la délivrance de lumière UV à partir de la première source de lumière comprend en outre :
une délivrance de la lumière UV lorsque le robot mobile (100) se déplace dans la trajectoire ou lorsque le robot est stationnaire.

10. Procédé (10) selon une quelconque revendication précédente, dans lequel la délivrance de la lumière UV à partir de la première source de lumière comprend en outre :
une délivrance de la lumière UV à partir de la première source de lumière sur l'au moins une partie des roues et la surface sur laquelle le robot mobile (100) se déplace lorsque le robot mobile (100) se déplace vers une station d'accueil ou se déplace entre une première zone et une seconde zone.

11. Procédé (10) selon une quelconque revendication précédente, dans lequel la délivrance de lumière UV à partir de la première source de lumière comprend :
un ajustement, par un dispositif de commande du robot mobile (100), d'une orientation de la première source de lumière de sorte que la première source de lumière délivre une lumière UV sur au moins une partie d'une de la pluralité de roues.
